(19) Europäisches Patentamt
European Patent Office
Office européen des brevets

(11) Numéro de publication: **0 031 786**
**B1**

# (12) FASCICULE DE BREVET EUROPEEN

(45) Date de publication du fascicule du brevet: **11.09.85**

(51) Int. Cl.⁴: **G 01 N 33/96**

(21) Numéro de dépôt: **80420149.9**

(22) Date de dépôt: **30.12.80**

(54) **Etalon de contrôle de liquide synthétique pour la vérification de la qualité des instruments de dosage des gaz dans le sang.**

(30) Priorité: **31.12.79 US 108480**

(43) Date de publication de la demande: **08.07.81 Bulletin 81/27**

(45) Mention de la délivrance du brevet: **11.09.85 Bulletin 85/37**

(84) Etats contractants désignés: **DE FR GB IT**

(56) Documents cités:
**EP - A - 0 016 633**
**FR - A - 2 408 838**
**US - A - 3 876 375**
**US - A - 3 973 913**
**US - A - 4 001 142**
**US - A - 4 199 471**

**CLINICAL CHEMISTRY, vol. 25, no. 3, mars 1979 EASTON, Pa. (US) W.T. POPE et al.: "An evaluation of ethylene glycol-based liquid specimens for use in quality control" pages 413-418**

(73) Titulaire: **Louderback, Allan Lee, 9661 Longden Avenue, Temple City California 91780 (US)**
Titulaire: **Szatkowski, Paul R., 24 Winthrop Road, Bethel Connecticut 06801 (US)**

(72) Inventeur: **Louderback, Allan Lee, 9661 Longden Avenue, Temple City California 91780 (US)**
Inventeur: **Szatkowski, Paul R., 24 Winthrop Road, Bethel Connecticut 06801 (US)**

(74) Mandataire: **Dupuis, François, Cabinet Charras 3 Place de l'Hôtel-de-Ville, F-42000 St.Etienne (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



BUNDESDRUCKEREI BERLIN

## Description

L'invention se rattache au secteur des moyens de détermination du pH dans le sang et divers autres paramètres comme procédure de diagnostic utilisée avec certains traitements médicaux et chirurgicaux.

La détermination du pH, de la pression partielle de gaz carbonique ($pCO_2$) et de la pression partielle d'oxygène ($PO_2$) dans le sang, est une procédure de diagnostic bien établie, utilisée en liaison avec certains traitements médicaux et chirurgicaux. Il est important de connaître la condition de circulation sanguine du malade en termes de son équilibre acide-base, des electrolytes et de ses niveaux de gaz dans le sang, à tous moments, au cours de ces traitements.

Différents instruments ont été étudiés et mis au point pour déterminer les paramètre comprenant l'équilibre acide-base et le dosage des gaz dnas le sang. Ces instruments ou appareils sont généralement aptes à mesurer le pH, le $pCO_2$ et le $pO_2$ du sang. Des exemples d'appareils sont décrits dans les Brevets U.S. 3 658 478, 3 652 843, 3 763 422; 3 654 445 et 3 874 850. On trouve dans le commerce certains appareils, dont le Corning 175 Automatic pH/Blood Gas System; le ABLI Acid Base Laboratory de la London Company, Radiometer Copenhague, le IL 113 pH/Blood Gas Analyser de Instrumentation Laboratory; et le Technicien BGII pH/Blood Gas System.

Il est de pratique courante d'employer des solutions contrôle pour vérifier la précision et la fiabilité de ces appareils. On peut citer à titre d'exemple, les solutions contrôles utilisées dans ces appareils, telles que les étalons de contrôle décrits dans les brevets US 3 973 913 et 4 126 575. Ces compositions contiennent des globules rouges de sang stabilisés dans une solution tampon de ions bicabonate avec un espace supérieur gazeux approprié, encapsulé dans un réceptacle étanche.

Un autre étalon de contrôle gaz sang est divulgué dans le brevet US 4 001 142. Cet étalon de contrôle décrit un contrôle de gaz dans le sang, de type synthétique qui manque d'éléments protéinacés. Ce document enseigne une composition nettement différente de celle revendiquée dans la demande. Elle mentionne l'utilisation d'un agent tampon triéthanolamine, en combinaison avec un acide d'alcoyle inférieur comme de l'acide acétique (qui ne sont pas utilisés dans la composition de gaz du sang selon la demande). De plus, ce brevet ne fait aucune mention ou suggestion d'éthylène glycol soit à des fins cryoprotectrices (comme indiqué dans la technique précédente) soit pour assurer la viscosité et le dégagement des proéines et favoriser la clarification de la matière protéinacée des systèmes d'instrumentation.

Une variante de ce type d'étalon de contrôle gaz sang décrit dans le brevet US 4 001 142, est définie dans la publication de la Demande de Brevet Allemand n° 2 727 140 et dans le brevet US 4 163 734, et comprend une solution aqueuse contenant un tampon et une teinture (Ponceau 4R) émulsifiés avec de la perfluorotributylamine et comporte des gaz dissous en équilibre avec ces produits.

Les instruments ou appareils précités existant dans la situation technique actuelle, emploient généralement des électrodes pour la mesure du pH, du $pCO_2$ et du $pO_2$. C'est ainsi par exemple, que la concentration en ions hydrogène peut être contrôlée avec une éléctrode en verre réagissant au pH, en collaboration avec une électrode de référence Ag/AgCL; la pression partielle de gaz carbonique ($pCO_2$) peut être captée dans le liquide de circulation, avec une électrode $pCO_2$, et la pression partielle d'oxygène ($pO_2$) peut, de façon similaire, être contrôlée avec une électrode d'oxygène. Ces électrodes de captage des gaz sont prévues avec une membrane sélectivement perméable par dessus la pointe, afin de permettre la passage du gaz perinent à capter. Ces membranes peuvent être en matériaux tels que du caoutchouc siliconé, du plastique vendu dans le commerce sous la marque »TEFLON«, et matériaux perméables similares, perméables au gaz mais imperméables au liquide.

Dans l'utilisation pratique, au bout d'un certain laps de temps, on constate que la matière protéine des échantillons de sang appliqués sur l'instrument de mesure des gaz dans le sang, a tendance à s'accumuler sur la membrane de l'electrode et à engorger ainsi les pores de la matière constituant la membrane semi-perméable ou à contaminer d'une autre façon les tubes des instruments.

Selon la présente invention, les Demandeurs ont mis au point un étalon de contrôle de liquide entièrement synthétique, utilisable dans les instruments de dosage des gaz du sang, qui assure un contrôle approprié tout en favorisant en même temps l'enlèvement de la matière protéine sur la membrane de l'électrode et sur les tubes.

Selon l'invention, l'étalon de contrôle de liquide synthétique utilisé pour la vérification de la qualité des instruments de dosage des gaz dans le sang, comprenant une solution aqueuse de teinture tamponnée à un pH de 7,1 à 7,7 et contenant suffisamment de ions bicarbonate pour assurer un $pCO_2$ de 20 à 80 de glycol éthylénique et d'oxygène gazeux pour un $PO_2$ de 50 à 400 contenue dans éthylénique pour assurer une viscosité de 3 à 9 mPa s (3 à 9 centipoises) pour favoriser la clarification de la matière protéinacée des systèmes d'instrumentation.

Selon l'invention, la méthode de préparation d'un étalon de contrôle de liquide synthétique pour utilisation dans la vérification de la qualité de systèmes d'instruments de dosage des gaz dans le sang et ayant une viscosité ressemblant à celle du sang, comprend la formation d'une solution aqueuse de teinture tamponnée à un pH de 7,1 à 7,7 et contenant suffisamment de ions bicarbonate pour assurer un $pCO_2$ de 20 à 80, de glycol éthylénique pour assurer une viscosité de 3 à 9 mPa s (3 à 9 centipoises), et d'oxygène gazeux pour assurer un $PO_2$ de 50 à 400, contenue dans un réceptacle hermétique.

L'étalon de contrôle selon la présente invention prévoit ainsi une composition synthétique quie ne contient pas de matière biologique naturelle telle que des globules de sang ou des composants de plasma ou de sérum sanguin protéinacés. Néanmoins, cette composition a une viscosité se rapprochant de celle du sang, ou similaire, et peut par conséquent, s'écouler dans les tubes des appareils de manière semblable à celle du sang normal, contrairement à l'eau ou aux solutions aqueuses usuelles de contrôle des gaz du sang. Etant donné que les instruments de mesure des gaz dans le sang sont prévus avec un système vide pour l'aspiration des échantillons de sang, pour les analyses des gaz du sang appropriées, l'étalon de contrôle de liquide selon la présente invention qui a une viscosité se rapprochant de celle du sang, ou similaire, s'écoulera dans les tubes des instruments d'une façon rigoureusement similaire au sang. Ceci assurera de ce fait, un meilleur étalon de contrôle et une représentation plus précise du sang normal q'un étalon de contrôle de liquide moins visqueux.

La viscosité est usuellement exprimée en dyne-secondes par centimètres carré ou en poises. Un poise équivaut à 100 centipoises. La viscosité absolue de l'eau à 20 degrés centigrades pour des applications de calibrage, est de 0,01002 poise, selon ce qui est rapporté par Swindelles et al, Journal of Research, National Bureau of Standards, 48, 1 (1952). Le sang a une viscosité qui est environ 5 à 6 fois plus élevée que celle de l'eau, soit environ 5 à 6 centipoises.

Le glycol éthylénique a, à 20 degrés centigrades, une viscosité de 19,9 centipoises. L'incoroporation d'environ 15 à 45 pour cent en volume de glycol éthylénique dans la solution aqueuse, assure ainsi la viscosité désirée de 3 à 9 centipoises environ pour la solution. La présence de ladite quantité de glycol éthylénique dans la solution aqueuse permet également à la solution de contrôle, de favoriser l'enlèvement de la matière protéine sur la surface de la membrane d'électrode et sur le système de tubes des instruments, lorsque la solution étalon de contrôle est appliquée sur l'instrument. En effet, le glycol éthylénique réduit la tension de surface ou la tendance des contaminants protéiniques à coller sur la surface de la membrane ou sur les tubes. Cette propriété de réduction de tension de surface du glycol éthylénique, aide également à l'éloignement de la teneur totale en étalon de contrôle de liquide à partir du réceptacle dans lequel il est contenu, et plus spécialement de cette partie de la solution qui a tendance à coller sur le haut d'une ampoule de verre cassable. Dans la pratique, l'ampoule contenant l'échantillon de l'étalon de contrôle est secouée avant l'utilisation, afin d'assurer un équilibre amélioré du liquide et des gaz dissous. Cette façon de procéder a tendance à faire coller une certaine partie de l'échantillon à la partie supérieure de l'ampoule. Etant donné que le haut de l'ampoule est enlevé, le reste de la solution dans l'ampoule sera inférieur à sa quantité spécifiée, ce qui est susceptible d'entraîner des inexactitudes dans sa mesure, lorsqu'il est appliqué sur l'instrument.

L'étalon de contrôle selon la présente invention contient une teinture appropriée pour indiquer à l'utilisateur l'équilibre acide-base relatif ou la condition métabolique gaz sang devant être représenté ou représenté ou représentée par l'étalon de contrôle, par exemple des conditions du sang normales, acidosiques, alcalosiques ou hyper-oxygénées.

A titre d'exemple, un étalon de contrôle gaz sang normal avec un pH d'environ 7,4, un $pCO_2$ d'environ 40 et un $pO_2$ d'environ 100, peut être repéré par une solution de teinture jaune. De préférence, on utilise 100 milligrammes environ de jaune FD & C N° 5 (C. I. 19 140; (tartrazine) dans 500 millilitres environ de solution aqueuse pour l'étalon de contrôle de sang normal. La condition métabolique acidosique avec un pH d'environ 7,2, und $pCO_2$ d'environ 20 et un $pO_2$ d'environ 150, peut être indiquée par une solution de teinture rouge. De préférence, on utilise 100 milligrammes environ de Rouge FD & C N° 40 (C. I. 16 035) dans 500 millilitres environ de solution aqueuse, pour l'étalon de contrôle de sang acidosique. La condition métabolique alcalosique avec un pH d'environ 7,6, un $pCO_2$ d'environ 60 et un $pO_2$ d'environ 60, peut être indiquée par une solution de teinture bleue. De préférence, on utilise 100 milligrammes environ de Bleu Peacock (C. I. 42 090 : 1) dans 500 millilitres environ de solution aqueuse, pour l'étalon de contrôle de sang alcalosique. La condition gaz sang hyper-oxygénée avec un pH normal d'environ 7,4 et un $pCO_2$ d'environ 60 mais présentant un $pO_2$ élevé d'environ 400, peut être indiquée par une solution de teinture verte. La solution de teinture verte s'obtient de préférence en combinant environ 50 milligrammes de Jaune FD & C N° 5 avec environ 50 milligrammes de Bleu Peacock dans 500 millilitres environ de solution aqueuse. D'autres solutions de teinture représentatives de ce genre, permettant d'assurer des distinctions colorées appropriées pour les différentes conditions de la pression des gaz dans le sang rencontrées chez les malades, se présenteront à l'esprit du technicien qualifié dans la branche, et il est bien entendu que l'invention n'est pas limitée aux exemples illustratifs ci-avant des teintures et solutions de teinture.

Afin d'assurer le pH désiré pour les conditions, respectivement normales, acidosiques ou alcalosiques, il faut choisir un produit tampon ayant un $pk_a$ se rapprochant du pH de régine désiré. Un produit tampon particulièrement utile pour assurer les conditions de pH désirées dans la solution de contrôle selon la présente invention, est l'acide N-2-hydroxyethylpiperazine-N'-2-éthanesulfonique (HEPES), qui a un $pK_a$ de 7,55 à 20 degrés centigrades. D'autres produits tampon appropriés sont par exemple: l'acide N-tris(hydroxymethyl)methyl-2-aminoéthanesulfonique (TES), qui a un $pk_a$ de 7,50 à 20 degrés centigrades; l'acide N,N-bis(2-hydroxyethyl)-2-aminoéthanesulfonique (BES), qui a un $pk_a$ de 7,15 à 20 degrés centigrades; l'acide 3-(N-morpholino)propanesulfonique (MOPS), qui a un $pk_a$ de 7,20 à 20 degrés centigrades; et l'acide Piperazine-N-N'-bis(2-éthanesulfonique) (PIPES), qui a un $pk_a$ de 6,8 à 20 degrés centigrades. Ces produits tampons appropriés, ainsi que d'autres, incluant les dérivés de sel de sodium, sont décrits par Good et al, Biochemistry 5, 467—77 (1966).

Le niveau désiré de $pCO_2$ est assuré en partie par addition de ions bicarbonate, par exemple du $NaHCO_3$ à la solution aqueuse jusqu'à ce qu'un $pCO_2$ de 20 à 80 environ soit obtenu. Le niveau de $pO_2$ désiré, de 50 à 400 environ, s'obtient plus facilement en ajoutant de l'oxygène gazeux à la solution ou dans l'espace supérieur, dans le réceptacle contenant la solution aqueuse. Similairement, l'addition de gaz carbonique gazeux peut faciliter le maintien des niveaux de $pCO_2$ désirés précités.

La solution étalon de contrôle finale est obtenue dans un réceptacle hermétique ou étanche à l'air, tel que par exemple, un flacon ou une ampoule de verre, de manière à conserver l'équilibre de gaz désiré. L'espace supérieur dans le réceptacle peut être rempli avec un gaz approrieé, afin de faciliter l'obtention des conditions de $pCO_2$ et de $pO_2$ précitées. Par exemple pour le contrôle des gaz du sang dans les conditions acidosiques, on utilise de préférence de l'air ambiant normal (environ 21 pour cent d'oxygène et 78 pour cent d'azote). Pour le contrôle des gaz du sang dans des conditions normales, on utilise de préférence un mélange d'environ 5 pour cent de gaz carbonique, 12 pour cent d'oxygène et 83 pour cent d'azote. Pour le contrôle des gaz du sang dans des conditions alcalosiques, on utilise de préférence un mélange d'environ 7 pour cent de gaz carbonique, 7 pour cent d'oxygène et 86 pour cent d'azote. Pour le contrôle des gaz du sang dans des conditions hyper-oxygénées, on utilise de préférence un mélange d'environ 40—60 pour cent d'oxygène et 40—60 pour cent d'azote. Il est bien entendu que tout autre gaz inerte peut être utilisé pour le remplacement en tout ou partie de la fraction azote de l'espace supérieur, dans les exemples illustratifs qui viennent d'être donnés ci-avant.

L'exemple spécifique et détaillé qui suit illustrera complémentairement l'invention, mais il est bien entendu que cet exemple n'est pas destiné à limiter l'invention aux détails spécifiques qui sont indiqués dans ledit exemple.

## Exemple

On prépare une solution tampon de manière que cette solution comprenne une solution millimolaire 150 de HEPES, en faisant dissoudre 35 745 grammes de HEPES dans 800 millilitres d'eau déionisée. On ajoute du NaOH 4N en quantité suffisante pour régler le pH à 7400. On ajoute ensuite du glycol éthylénique dans une quantité de 200 millilitres, de façon à amener le volume total à 1000 millilitres et à obtenir une solution tampon/glycol contenant du tampon HEPES 150 mM à un pH de 7400 (à une température ambiante d'environ 22—25 degrés centigrades), dans une solution à 20 pour cent de glycol éthylénique. On prépare de la même manière un autre litre de la solution tampon/glycol, afin de disposer de deux litres pour cet exemple.

On prépare ensuite quatre solutions aqueuses teinture/tampon/glycol, séparées, en faisant dissoudre trois teintures basiques dans des solutions tampon/glycol telles que celles préparées ci-dessus, dans les proportions suivantes:

| | | |
|---|---|---|
| Acidosique | — | solution rouge — 100 milligrammes de Rouge FD & C N° 40 dans 50 millilitres de la solution tampon/glycol |
| Normale | — | solution jaune — 100 milligrammes de Jaune FD & C N° 5 dans 500 millilitres de la solution tampon/glycol |
| Alcalosique | — | solution bleue — 100 milligrammes de Bleu Peacock dans 500 millilitres de la solution tampon/glycol |
| Hyperoxygénée | — | solution verte — 50 milligrammes de Bleu Peacock et 50 milligrammes de Jaune FD & C dans 500 millilitres de la solution tampon/glycol. |

Etant donné que les instruments de dosage des gaz dans le sang existant actuellement dans la situation technique, sont généralement prévus pour mesurer à 37 degrés centigrades plutôt qu'à la température ambiante normale, on règle les pH des solutions précitées teinture/tampon/glycol, aux niveaux désirés (acidosique — pH 7,2; normale — pH 7,4; alcalosique pH 7,6; et hyper-oxygénée — pH 7,4), par addition de HCl 1N ou NaOH 1N, selon ce qui est nécessaire pour concorder avec le pH des instruments à 37 degrés centigrades.

On ajoute ensuite une solution de $NaHCO_3$ 1M aux solutions teinture/tampon/glycol par petites fractions, jusqu'à ce que les niveaux de $pCO_2$ désirés soient atteints, comme suit:

| | |
|---|---|
| Acidosique | $pCO_2$ 20 |
| Normal | $pCO_2$ 40 |
| Alcalosique | $pCO_2$ 60 |
| Hyperoxygénée | $pCO_2$ 40. |

Puis on remplit avec les solutions préparées ci-avant, des ampoules de verre du type à partie supérieure cassable (capacité 5 millilitres chacune). Chaque ampoule est remplie jusqu'à un niveau de 2 millilitres. L'espace supérieur dans l'ampoule est ensuite garni avec les mélanges gazeux désirés, comme suit:

| | | |
|---|---|---|
| Acidosique | — | air ambiant normal se composant d'environ 21 pour cent d'oxygène et 78 pour cent d'azote |
| Normal | — | pour cent de gaz carbonique, 12 pour cent d'oxygène et 83 pour cent d'azote |
| Alcalosique | — | 7 pour cent de gaz carbonique, 7 pour cent d'oxygène et 86 pour cent d'azote |
| Hyperoxygéné | — | 60 pour cent d'oxygène et 40 pour cent d'azote. |

Les ampoules sont ensuite scellées à la flamme de façon à obtenir une fermeture étanche à l'air, et le produit conditionné est stocké à la température ambiante normale (environ 22—25 degrés centigrades). Le produit final a une viscosité d'environ 5 à 6 centipoises, et lorsqu'il est appliqué sur des instruments de dosage des gaz du sang, il s'écoule d'une façon semblable en viscosité, au sang normal, et empêche, sur les tubes et membranes desdits instruments, l'accumulation de la protéine provenant des échantillons de sang appliqués sur les instruments, et la contamination qui en résulte. Le produit final peut être stocké en permanence à la température ambinante normale, et il n'est pas nécessaire de le stocker à des températures réfrigérées telles que celles de l'ordre de 2 à 8 degrés centigrades qui sont requises par les compositions biologiques décrites dans les Brevets U.S. N° 3 876 375 et 4 121 905. Le glycol dans l'étalon de contrôle de la présente invention, agit également comme stabilisateur et comme préservatif, et permet par conséquent, d'éviter la mise en autoclave autrement requise pour la stérilité, comme on peut s'en rendre compte d'après le Brevet U.S 4 001 142.

Des résultats rigoureusement similaires à l'exemple ci-avant sont obtenus losque les solutions teinture/tampon/glycol sont réglées sur les niveaux de pH suivants:

| | |
|---|---|
| Acidosique | pH 7,1 à 7,3 |
| Normal | pH 7,31 à 7,5 |
| Alcalosique | pH 7,51 à 7,7 |
| Hyperoxygéné | pH 7,31 à 7,5 |

Dans l'exemple ci-dessus, les niveaux finals de $pO_2$ sont comme suit:

| | |
|---|---|
| Acidosique | $pO_2$ 150 |
| Normal | $pO_2$100 |
| Alcalosique | $pO_2$ 60 |
| Hyperoxygéné | $pO_2$ 400 |

D'autres réglages appropriés en $pO_2$ et $pCO_2$ peuvent être effectués en fonction de ce qu'on désire dans les limites des grammes divulguées. C'est ainsi par exemple que dans le contrôle des gaz-sang hyperoxygéné, le $pO_2$ peut être réglé sur d'autres niveaux, dans une gamme préférée de 200 à 400, et que le $pCO_2$ peut être réglé sur d'autres niveaux dans une gamme préférée de 20 à 60, avec des résultats rigoureusement similaires à ceux obtenus dans l'exemple ci-avant.

D'autres exemples encore, après lecture de la présente description, apparaitront de façon évidente aux spécialistes de la partie, sans que ces exemples s'écartent pour autant de l'esprit et de la portée de l'invention, et il est bien entendu que tous exemples de ce genre sont inclus dans le cadre des revendications ci-annexées.

**Revendications**

1. Etalon de contrôle de liquide synthétique utilisé pour la vérification de la qualité des instruments de dosage des gaz dans le sang; comprenant une solution aqueuse de teinture tamponnée à un pH de 7,1 à 7,7 et contenant suffisamment de ions bicarbonate pour assurer un $pCO_2$ de 20 à 80, de glycol éthylénique et d'oxygène gazeux pour un $PO_2$ de 50 à 400 contenue dans un réceptacle hermétique caractérisé en ce qu'il comprend du glycol éthylénique pour assurer une viscosité de 3 à 9 m Pa s (3 à 9 centipoises) pour favoriser la clarification de la matière protéinacée des systèmes d'instrumentation.

2. Etalon de contrôle selon 1, caractérisé en ce que la solution aqueuse est tamponnée avec un agent tampon acide N-2-hydroxyethylpiperazine-N'-2-éthanesulfonique.

3. Etalon de contrôle selon 1, caractérisé en ce que le glycol éthylénique assure une viscosité de 5 à 6 m Pa s (5 à 6 centipoises).

4. Etalon de contrôle selon 1, caractérisé en ce que la teinture est choisie dans un groupe se composant de Rouge FD & C N° 40, (C.I.16035) de jaune FD & C N° 5, (C.I. 19140) de Bleu Peacock (C.I. 42090 : 1) et de mélanges desdits jaunes FD & C N° 5 et Bleu Peatock.

5. Etalon de contrôle selon 1, caractérisé en ce que la solution aqueuse est tamponnée avec l'acide N-2-hydroxyethylpiperazine-N'-2-éthanesulfonique, le glycol éthylénique assurant une viscosité de 5 à 6 m Pa s (5 à 6 centipoises),la teinture étant du Rouge FD & C N° 40, le pH étant de 7,2, le $pCO_2$ étant de 20 et $PO_2$ étant de 150.

6. Etalon de contrôle selon 1, caractérisé en ce que la solution aqueuse est tamponnée avec de l'acide N-2-Hydroxyethylpiperazine-N'-2-éthanesulfonique, le glycol éthylénique assurant une viscosité de 5 à 6 m Pa s (5 à 6 centipoises), la teinture étant du jaune FD & C N° 5, le pH étant de 7,4, le $pCO_2$ étant de 40 et le $PO_2$ étant de 100.

7. Etalon de contrôle selon 1, dans lequel la solution aqueuse est tamponnée avec de l'acide N-2-hydroxyethylpiperazine-N'-2-éthanesulfonique, le glycol éthylénique assurant une viscosité de 5 à 6 m Pa s (5 à 6 centipoises), la teinture étant du Bleu Peacock, le pH étant de 7,6, le $pCO_2$ de 60 et le $PO_2$ étant de 60.

8. Etalon de contrôle selon 1, dans lequel la solution aqueuse est tamponnée avec l'acide N-2-hydroxyethylpiperazine-N'-2-éthanesulfonique, le glycol éthylénique assurant une viscosité de 5 à 6 m Pa s (5 à 6 centipoises), la teinture étant un mélange de jaune FD & C N° 5 et de Bleu Peacock; le pH étant de 7,4, le $pCO_2$ étant de 40 et le $PO_2$ étant de 400.

9. Méthode de préparation d'un étalon de contrôle de liquide synthétique pour utilisation dans la vérification de la qualité de systemès d'instruments de dosage des gaz dans le sang et ayant une viscosité ressemblant à celle du sang, comprenant la formation d'une solution aqueuse de teinture tamponnée à un pH de 7,1 à 7,7 et contenant suffisamment de ions bicarbonate pour assurer un $pCO_2$ de 20 à 80, de glycol éthylénique pour assurer une viscosité de 3 à 9 m Pa s (3 à 9 centipoises), et d'oxygène gazeux pour assurer un $PO_2$ de 50 à 400, contenue dans un réceptacle hermétique.

10. Etalon de contrôle selon 1, dans lequel le réceptacle comprend un espace supérieur rempli avec un mélange gazeux comprenant de l'oxygène et de l'azote.

11. Etalon de contrôle selon 10, dans lequel le mélange gazeux comprend du gaz carbonique.

**Patentansprüche**

1. Kontrollstandard für die zur Prüfung der Güte der Geräte für die Dosierung der Gase in dem Blut benutzte Kunstflüssigkeit, der eine auf einem pH-Wert von 7,1 bis 7,7 eingestellte wässerige Farbstofflösung aufweist, mit genug Doppelkarbonat-Ionen für einen $pCO_2$-Wert von 20 bis 80, genug Äthylenglykol und genug gasförmigem Sauerstoff für einen $PO_2$-Wert von 50 bis 400, die in einem hermetischen Gefäß enthalten ist, dadurch gekennzeichnet, daß dieser Kontrollstandard genug Äthylenglykol für eine Zähigkeit von 3 bis 9 m Pa s (3 bis 9 Centipoisen) aufweist, damit die Klärung des Proteinstoffs der Gerätesysteme befördert wird.

2. Kontrollstandard nach Anspruch 1, dadurch gekennzeichnet, daß die wässerige Lösung mit einem aus N-2-Hydroxyäthylpiperazine-N'-2-äthansulfonsäure bestehenden pH-Wertregler auf den vorbestimmten pH-Wert eingestellt wird.

3. Kontrollstandard nach Anspruch 1, dadurch gekennzeichnet, daß eine Zähigkeit von 5 bis 6 m Pa s (5 bis 6 Centipoisen) aus dem Äthylenglykol entsteht.

4. Kontrollstandard nach Anspruch 1, dadurch gekennzeichnet, daß der Farbstoff aus einer Gruppe gewählt wird, die aus FD & C Rot Nr. 40 (C.I. 16035), aus FD & C Gelb Nr. 5 (C.I. 19140), aus Peacock-Blau und aus Mischungen von den besagten FD & C Geldfarben und von Peacock-Blau besteht.

5. Kontrollstandard nach Anspruch 1, dadurch gekennzeichnet, daß die wässerige Lösung mit der N-2-Hydroxyäthylpiperazine-N'-2-äthansulfonsäure auf den vorbestimmten pH-Wert eingestellt wird, daß aus dem Äthylenglykol eine Zähigkeit von 5 bis 6 m Pa s (5 bis 6 Centipoisen) entsteht, und daß der Farbstoff aus FD & C Rot Nr. 40 besteht, wobei die folgenden Werte erreicht werden: pH-Wert 7,2; $pCO_2$ 20; und $PO_2$ 150.

6. Kontrollstandard nach Anspruch 1, dadurch gekennzeichnet, daß die wässerige Lösung mit der N-2-Hydroxyäthylpiperazine-N'-2-äthansulfonsäure auf den vorbetimmten pH-Wert eingestellt wird, daß aus dem Äthylenglykol eine Zähigkeit von 5 bis 6 m Pa s (5 bis 6 Centipoisen) entsteht, und daß der Farbstoff aus FD & C Gelb Nr. 5 besteht, wobei die folgenden Werte erreicht werden: pH-Wert 7,4; $pCO_2$ 40; und $PO_2$ 100.

7. Kontrollstandard nach Anspruch 1, dadurch gekennzeichnet, daß die wässerige Lösung mit der N-2-Hydroxyäthylpiperazine-N'-2-äthansulfonsäure auf den vorbestimmten pH-Wert eingestellt wird, daß aus dem Äthylenglykol eine Zähigkeit von 5 bis 6 m Pa s (5 bis 6 Centipoisen) entsteht, und daß der Farbstoff aus Peacock-Blau besteht, wobei die folgenden Werte erreicht werden: pH-Wert 7,6; $pCO_2$ 60 und $PO_2$ 60.

8. Kontrollstandard nach Anspruch 1, dadurch gekennzeichnet, daß die wässerige Lösung mit der N-2-Hydroxyäthylpiperazine-N'-2-äthansulfonsäure auf den vorbestimmten pH-Wert eingestellt wird, daß aus dem Äthylenglykol eine Zähigkeit von 5 bis 6 m Pa s (5 bis 6 Centipoisen) entsteht, und daß der Farbstoff aus einer Mischung von FD & Gelb Nr. 5 und Peacock-Blau besteht, wobei die folgenden Werte erreicht werden: pH-Wert 7,4; $pCO_2$ 40, und $PO_2$ 400.

9. Verfahren zur Bereitung eines Kontrollstandards für die zur Prüfung der Güte von Gerätesystemen für die Dosierung der Gase in dem Blut benutzte Kunstflüssigkeit, die eine der Blutzähigkeit ähnliche Zähigkeit hat, mit Bildung einer auf einen pH-Wert von 7,1 bis 7,7 eingestellten wässerigen Farbstofflösung, wobei diese Lösung genug Doppelkarbonat-Ionen für einen $pCO_2$-Wert von 20 bis 80, genug Äthylenglykol für eine Zähigkeit von 3 bis 9 m Pa s (3 bis 9 Centipoisen), und genug gasförmigem

Sauerstoff für einen $PO_2$-Wert von 50 bis 400 enthält und in einem hermetischen Gefäß enthalten ist,

10. Kontrollstandard nach Anspruch 1, dadurch gekennzeichnet, daß das Gefäß einen Oberraum aufweist, der mit einer gasförmigen Mischung von Sauerstoff und Stickstoff gefüllt ist.

11. Kontrollstandard nach Anspruch 10, dadurch gekennzeichnet, daß Kohlengas in der gasförmigen Mischung enthalten ist.

**Claims**

1. Control standard of synthetic liquid used for acertaining the quality of instruments for the dosage of gas in the blood, comprising an aqueous tincture solution buffered at a pH of 7.1 to 7.7 and containing a quantity of bicarbonate ions sufficient to provide a $pCO_2$ of 20 to 80 of ethylene glycol and gazeous oxygen for a $PO_2$ of 50 to 400 contained in a sealed vessel, characterized in that said standard includes the ethylene glycol to provide a viscosity of 3 to 9 m Pa s (3 to 9 centipoises) in order to promote the clarification of the proteinaceous material of the instrumentation systems.

2. Control standard as claimed in Claim 1, characterized in that the aqueous solution is buffered with a N-2-hydroxyethylpiperazine-N'-2-ethanesulfonic acid buffer agent.

3. Control standard as claimed in Claim 1, characterized in that the ethylene glycol provides a viscosity of 5 to 6 m Pa s (5 to 6 centipoises).

4. Control standard as claimed in Claim 1, characterized in that the tincture is selected within a group consisting of FD & C Red N° 40 (C.I. 16035), of FD & C Yellow N° 5 (C.I. 19140), of Peacock Blue (C.I. 42090 : 1), and of mixtures of said FD & C Yellow N° 5 and said Peacock Blue.

5. Control standard as claimed in Claim 1, characterized in that the aqueous solution is buffered with the N-2-hydroxyethylpiperazine-N'-2-ethanesulfonic acid, the ethylene glycol providing a viscosity of 5 to 6 m Pa s (5 to 6 centipoises), the tincture being the FD & C Red N° 40, the pH being 7.2, the $pCO_2$ being 20, and the $PO_2$ being 150.

6. Control standard as claimed in Claim 1, characterized in that the aqueous solution is buffered with the N-2-hydroxyethylpiperazine-N'-2-ethanesulfonic acid, the ethylene glycol providing a viscosity of 5 to 6 m Pa s (5 to 6 centipoises), the tincture being the FD & C Yellow N°5, the pH being 7.4, the $pCO_2$ being 40, and the $PO_2$ being 100.

7. Control standard as claimed in Claim 1, in which the aqueous solution is buffered with the N-2-hydroxyethylpiperazine-N'-2-ethanesulfonic acid, the ethylene glycol providing a viscosity of 5 to 6 m Pa s (5 to 6 centipoises), the tincture being the Peacock Blue, the pH being 7.6, the $pCO_2$ being 60, and the $PO_2$ being 60.

8. Control standard as claimed in Claim 1, in which the aqueous solution is buffered with the N-2-hydroxyethylpiperazine-N'-2-ethanesulfonic acid, the ethylene glycol providing a viscosity of 5 to 6 m Pa s (5 to 6 centipoises), the tincture being a mixture of FD & C Yellow and Peacock Blue, the pH being 7.4; the $pCO_2$ being 40 and the $PO_2$ being 400.

9. Method for preparing a control standard of synthetic liquid to be used in the vertification of the quality of the instrument systems for the dosage of the gases in the blood and having a viscosity similar to the viscosity of the blood, said method comprising the formation of a tincture aqueous solution buffered at a pH of 7.1 to 7.7 and containing a quantity of bicarbonate ions sufficient to provide a $pCO_2$ of 20 to 80, a quantity of ethylene glycol sufficient to provide a viscosity of 3 to 9 m Pa s (3 to 9 centipoises), and a quantity of gaseous oxygen sufficient to provide a $PO_2$ of 50 to 400, the solution being contained within a sealed container.

10. Control standard as claimed in Claim 1, in which the container includes an upper space filled with a gaseous mixture comprised of oxygen and azote.

11. Control standard as claimed in Claim 10, in which carbon dioxide is included in the gaseous mixture.